# EUROPEAN PATENT APPLICATION

(11) **EP 1 787 676 A1**
(43) Date of publication of application: **23.05.2007**
(21) Application number: 05781548.2
(22) Date of filing: 01.09.2005
(51) Int. Cl.: A61M 29/02

(54) **STENT FOR PLACEMENT IN BODY**

(30) Priority: 08.09.2004 JP 2004260793
(71) Applicant: Kaneka Corporation, Kita-ku Osaka-shi, Osaka 530-8288 (JP)
(72) Inventor: NISHIDE, Takuji, KANEKA CORPORATION, Settsu-shi, Osaka 566-0072 (JP)
(74) Representative: Gille Hrabal Struck Neidlein Prop Roos
(86) International application number: PCT/JP2005/016000
(87) International publication number: WO 2006/027992

(57) **Abstract**

Disclosed is an easy-to-manufacture stent for placement in a body having a coating layer composed of a polymer wherein separation or cracking of the coating layer due to expansion of the stent canbeefficientlyprevented. Specificallydisclosed is a stent for placement in a body which comprises a coating layer composed of a polymer and an intermediate layer arranged between the coating layer and the surface of the stent and composed of a polymer having a higher weight average molecular weight than the polymer of the coating layer. The polymers are preferably biodegradable, and able to contain a medical agent.

## Description

### TECHNICAL FIELD

The present invention relates to a medical stent for placement in body, for use in dilating stenosed blood vessel.

### BACKGROUND ART

One of the serious problems on health we face currently is blood vessel stenosis caused by arteriosclerosis. In particular, stenosis of cardiac coronary artery is known to lead to severe diseases such as angina pectoris and myocardial infarction, very frequently resulting in death. One of the methods for treatment of such a blood vessel stenosis site, which is widely practiced as a minimal Invasive Treatment, is angioplasty (PTA, PTCA) of dilating the stenosis site by expansion of a small balloon inserted into blood vessel. However, the angioplasty leads to repeated stenosis (restenosis) at high probability. Various treatments such as atrectomy, laser treatment, and radiation treatment were studied for reducing the frequency of restenosis (restenosis rate), and recently, a method of placing a stent is used more widely.

The stent is a medical device that is placed in a blood vessel or other lumen in the body for preservation of the lumen size, after dilation of the corresponding stenosed or occluded site when it is constricted or occluded. The stent is generally made of a metal, a polymer, or the composite thereof, and stents of a metal such as stainless steel are used most commonly.

In treatment with a stent, the stent is inserted into blood vessel with a catheter and expanded for mechanical support of the vascular lumen when it becomes in contact with the unhealthy region of vascular wall. Although the restenosis rate after treatment by such a stent placement method becomes statistically significantly smaller than that by angioplasty only with a balloon, it is still significantly high currently. For example in the case of cardiac coronary artery, the restenosis rate after stent placement treatment is reported to be as high as approximately 20 to 30%. The restenosis is said to be caused by excessive reaction for restoring the blood vessel physically damaged by stent placement, i.e., rapid neointimal hyperplasia, for example, by growth of smooth muscle cells in media after blood vessel damage, migration of the grown smooth muscle cells into intima, and migration of T cells and macrophages into the intima.

Recently for reduction of the restenosis rate after stent placement, proposed is a method of coating an antiocclusion drug on the stent. In Patent Document 1, drugs such as anticoagulant, antiplatelet, antibacterial, antitumor, antimicrobial, anti-inflammatory, antimetabolic, andimmunosuppressiveagents are studied as the antiocclusion drug. As for the immunosuppressive agent, cyclosporine, tacrolimus (FK506), sirolimus (rapamycin), mycophenolate mofetil, and the analogs thereof (everolimus, ABT-578, CCI-779, AP23573, etc.) are studied for reduction of the restenosis rate as they are coated on stent. For example, Patent Document 2 discloses a stent coated with an immunosuppressive agent sirolimus (rapamycin), while Patent Document 3 discloses a stent coated with an antitumor drug taxol (paclitaxel). Alternatively, Patent Documents 4 and 5 disclose a stent coated with tacrolimus (FK506).

Tacrolimus (FK506), compound having a CAS number of 104987-11-3, is disclosed, for example, in Patent Document 6. Tacrolimus (FK506), which is considered to inhibit mainly production of differentiation-growth factors, i.e. , cytokines such as IL-2 and INF-γ, in T cell by forming a complex with FK506-binding protein (FKBP) in the cell, is well known to be used as a preventive or treatment drug for prevention of rejection during organ transplantation and also for autoimmune diseases. Nonpatent Literature 1 confirms that tacrolimus (FK506) has an action to inhibit growth of human vascular cell.

As for the method of applying a medicine on stent, Patent Document 1 discloses use of a polymer, favorably a biodegradable polymer, as a carrier for the medicine. Patent Document 7 discloses use of a biodegradable polymer, and an example of the biodegradable polymer is polylactic acid.

In coating a polymer on stent, the coating method and the coating condition should be optimized for prevention of the exfoliation and cracking coating layer associated with stent expansion. The exfoliation and cracking of the coating layer, which frequently leads to severe disorders such as occlusion of blood vessel by excessive thrombus generation in the acute period after stent placement, are extremely dangerous. There is no description on typical methods of preventing such exfoliation and cracking in Patent Documents 1 and 7.

Patent Document 1: Japanese Unexamined Patent Publication No. 5-502179

Patent Document 2: Japanese Unexamined Patent Publication No. 6-009390

Patent Document3:Japanese Unexamined Patent Publication No. 9-503488

Patent Document 4: WO 02/065947

Patent Document 5: EP 1254674

Patent Document 6: Japanese Unexamined Patent Publication No. 61-148181

Patent Document 7: Japanese Unexamined Patent Publication No. 5-509008

Nonpatent Literature 1: Paul. J. Mohacsi MD, et al. The Journal of Heart and Lung Transplantation, May 1997 Vol. 16, No. 5, 484-491

### DISCLOSURE OF THE INVENTION

### Problems to be Solved by the Invention

An object of the present invention, which was made under the circumstances above, is to provide easily a stent for placement in body having a coating layer of polymer that is resistant to the exfoliation and cracking associated with expansion of the stent.

### Means to Solve the Problems

Accordingly, the present invention provides:
(1) An almost tube-shaped stent for placement in body expandable toward outside in the radial direction of the tube, characterized by including a stent main body containing a material non-degradable in the body as its base stent material, a coating layer containing a first polymer and a medicine, and an intermediate layer containing a second polymer having a weight-average molecular weight higher than that of the first polymer between the coating layer and the stent main body surface, wherein the coating layer and the intermediate layer are present on at least part of the stent main body surface;
(2) The stent for placement in body according to (1) wherein, the medicine is an immunosuppressive agent;
(3) The stent for placement in body according to (2) wherein, the immunosuppressive agent is at least one compound selected from tacrolimus (FK506), cyclosporine, sirolimus (rapamycin), azathioprine, mycophenolate mofetil and the analogs thereof;
(4) The stent for placement in body according to (3) wherein, the immunosuppressive agent is tacrolimus (FK506);
(5) The stent for placement in body according to any one of (1) to (4) wherein, the first and second polymers are respectively first and second biodegradable polymers;
(6) The stent for placement in body according to (5) wherein, each of the first and second biodegradable polymers is at least one polymer selected from polylactic acid, polyglycolic acid, and lactic acid-glycolic acid copolymers;
(7) The stent for placement in body according to (5) wherein, the biodegradation period of the first biodegradable polymer is shorter than that of the second biodegradable polymer; and
(8) The stent for placement in body according to any one of (1) to (7) wherein, the weight-average molecular weight of the first polymer is 10,000 or more and 50,000 or less and that of the second polymer is 80,000 or more and 200,000 or less.

### Advantageous Effects of the Invention

The stent for placement in body according to the invention is a stent containing a material non-degradable in the body as its base stent material, a coating layer containing a polymer as the principal component on the surface of the stent main body, and an intermediate layer of a polymer having a weight-average molecular weight of greater than that of the polymer above between the coating layer and the stent main body surface, which is effectively resistant to the exfoliation and cracking of the coating layer associated with stent expansion. In addition, the stent for placement in body according to the invention can be prepared more easily than conventional stents for placement in body.

### BRIEF DESCRIPTION OF THE DRAWINGS

Figure 1 is a development view of a stent.
Figure 2 is a schematic view of the stent.
Figure 3 is a SEM observation image obtained in Example 3.
Figure 4 is a SEM observation image obtained in Comparative Example 6.

### BEST MODE OF CARRYING OUT THE INVENTION

The present invention relates to an almost tube-shaped stent, comprising a stent main body that is expandable toward outside in the radial direction of the tube and contains a material non-degradable in the body as the base stent material, a coating layer containing a polymer and a medicine on at least part of the stent main body surface, and an intermediate layer of a polymer having a weight-average molecular weight higher than the polymer between the coating layer and the stent surface. The base stent material according to the present invention means a material for the stent having no coating layer. The stent main body can be prepared by cutting a base stent material, such as a tube-shaped material, into the stent shape, for example by laser cutting.

The coating layer and the intermediate layer are preferably formed on almost entire surface of the external, internal, and side faces of the stent main body. The stent main body having the coating and intermediate layers on the almost entire surface is thus resistant to deposition of platelet on the surface of the stent placed in a body lumen, in particular in blood vessel, possibly preventing the occlusion of blood vessel by generation of an excessive amount of thrombus.

The "material non-degradable in the body" for use in the present invention is a material not easily degradable biologically; thus, it is not a material that no decomposition occurs at all in the body, but a material that can keep its original shape relatively for an extended period of time; and such a material is also included in the "material non-degradable in the body" according to the invention.

Examples of the materials non-degradable in the body according to the present invention, i.e., base stent materials, include inorganic materials such as stainless steel, Ni-Ti alloys, Cu-Al-Mn alloys,tantalum,Co-Cr alloys,iridium,iridium oxide, niobium, ceramics, and hydroxyapatite. The stent main body can be prepared by a method normally practiced by those who are skilled in the art, and, for example, it can be prepared by cutting a tube-shaped material tube of the base stent material into the stent shape for example by laser cutting, as described above. The stent may be polished electrically after the laser cutting. The material non-degradable in the body according to the present invention is not limited to an inorganic material, and a polymeric material such as polyolefin, polyolefin elastomer, polyamide, polyamide elastomer, polyurethane, polyurethane elastomer, polyester, polyester elastomer, polyimide, polyamide-imide, or polyether ether ketone may be used. The method of producing a stent main body by using such a polymeric material does not restenose the advantageous effects of the present invention, and any processing method may be used arbitrarily according to the material used. The stent according to present invention, which contains a material non-degradable in the body as its stent base material, retains its favorable stent strength for a longer period of time and is extremely more effective in vasodilating the stenosed or occluded site of blood vessel than a stent having its stent main body made of a biodegradable material.

The stent main body surface preferably has at least partially a coating layer containing a polymer as the principal component and additionally a medicine and an intermediate layer of a polymer having a weight-average molecular weight of greater than that of the polymer above between the coating layer and the stent surface, and more preferably, the coating layer and the intermediate layer over the almost entire surface of the external, internal, and side faces of the stent main body. It is thus possible to reduce the exfoliation and cracking of the coating layer associated with stent expansion by forming the intermediate layer, and to prevent the exfoliation and cracking more effectively by making the weight-average molecular weight of the polymer for coating layer greater than that of the polymer for the intermediate layer.

The weight-average molecular weight of the polymer for the coating layer is preferably 10,000 or more and 50,000 or less, and the weight-average molecular weight of the polymer for the intermediate layer 80,000 or more and 200,000 or less. A coating layer of a polymer having a weight-average molecular weight of 10,000 or more and 50,000 or less, when it is formed directly on the stent main body surface, often results in cracking and exfoliation of the coating layer associated with stent expansion. If the coating layer of a polymer having a weight-average molecular weight of 10,000 or more and 50,000 or less contains a low-molecular weight compound such as medicine (molecular weight: ca. 5,000 or less), there are cracking and exfoliation of the coating layer associated with stent expansion at higher probability. In any case, it is possible to prevent exfoliation and cracking effectively by forming an intermediate layer between the stent main body surface and the coating layer and controlling the weight-average molecular weight of the polymer for the intermediate layer in the range of 80,000 or more and 200,000 or less.

Both of the polymers for the coating and intermediate layers are preferably biodegradable polymers. Generally if placement of the stent at bloodvessel stenosis site is considered, the degradation products from the biodegradable polymer should be completely degraded and metabolized safely in the body. From the viewpoint above, the biodegradable polymer is more preferably selected from polylactic acid, polyglycolic acid, and lactic acid-glycolic acid copolymers. Polylactic acids are available in three kinds of structures depending on the optical activity of the lactic acid monomer: poly-L-lactic acid, poly-D-lactic acid, and poly-D,L-lactic acid, but polylactic acid in any structure shows the advantageous effects of the present invention. Use of a biodegradable polymer results in disappearance of all polymer by biodegradation in the chronic phase after stent placement and in residual only of the stent base material in the body. It is possible to provide a stent higher in stability and reliability also in the chronic phase, easily by using a reliable metal material, such as SUS316L, as the stent base material.

The biodegradable polymers exemplified above have a glass transition temperature not lower than the body temperature, although it may vary according to the composition, and thus, are in the rigid glass state at around body temperature. In addition, poly-L-lactic acid, poly-D-lactic acid, polyglycolic acid, and the like are known to show high crystallinity. For that reason, the biodegradable polymers exemplified above show a tensile strength higher and a tensile breaking elongation shorter than other polymers such as thermoplastic elastomers. Thus, use of such a biodegradable polymer in forming a coating layer on the surface of the stent main body, caused a problem that there was an extremely high possibility of cracking and exfoliation of the coating layer associated with stent expansion. The possibility of cracking, exfoliation, and the like becomes even greater when the coating layer contains a medicine. However, it is possible to reduce the possibility of the cracking and exfoliation of the coating layer associated with stent expansion significantly and to coat the biodegradable polymer exemplified above favorably, by forming the intermediate layer according to the invention between the stent main body surface and the coating layer.

The biodegradation period of biodegradable polymer for the coating layer is preferably shorter than that of the biodegradable polymer for the intermediate layer. When each of the coating and intermediate layers is made of a biodegradable polymer, the coating layer and the intermediate layer disappears by biodegradation in the chronic phase after placement of a stent in body lumen. The intermediate layer has a function to improve the adhesiveness between the coating layer and the stent surface, and thus, the intermediate layer should not be biodegraded sooner than the coating layer.

The biodegradation period of the biodegradable polymer is calculated by using the change in weight, strength, or molecular weight of the biodegradable polymer as an indicator. Generally, the biodegradation period calculated from the molecular weight change is shortest, that calculated from the strength change is second shortest, and that calculated from the weight change is longest. The biodegradation period, independent of the indicator used for calculation, does not restenose the advantageous effects of the present invention. Because the biodegradation periods of a single biodegradable polymer calculated from different indicators are different from each other, as described above, the biodegradation period of the biodegradable polymer for the intermediate layer and that of the biodegradable polymer for the coating layer should be the values calculated from the same indicator.

The method of forming the intermediate layer and the coating layer on the stent main body surface is not particularly limited. In a favorable method, a polymer for the intermediate layer is dissolved in a solvent; the polymer in the solution state is applied on the surface of a stent main body, and the solvent is removed: a polymer for the coating layer is dissolved in a solvent; and the polymer in the solution state applied on the surface of the intermediate layer and the solvent is removed. Alternatively, a film of the polymer for the intermediate layer may be prepared separately and bonded to the stent main body, and a film of the polymer for the coating layer bonded to the surface of the intermediate layer. Yet alternatively, a polymer for the intermediate layer may be dissolved in a solvent; the polymer in the solution state is applied on the surface of a stent main body, and the solvent removed; and then, a film of a polymer for the coating layer be prepared separately and bonded to the surface of the intermediate layer, and yet alternatively, a film of a polymer for the intermediate layer may be prepared separately and bonded to the stent main body, a polymer for the coating layer dissolved in a solvent; and the polymer in the solution state applied on the surface of the the intermediate layer and the solvent removed

The coating layer contains a medicine for reduction of the restenosis rate after stent placement. The medicine is preferably an immunosuppressive agent, favorably tacrolimus (FK506), cyclosporine, sirolimus (rapamycin), azathioprine, mycophenolate mofetil or the analog thereof, more preferably tacrolimus (FK506).

The medicine may be added to the coating layer, for example, by preparing an intermediate layer by the method described above, dissolving a polymer and a medicine for the coating layer in a solvent, applying the solution on the surface of the intermediate layer in the solution state and removing the solvent, or by preparing a film of a polymer for the coating layer separately, coating the film with a solution of the medicine dissolved in a solvent by dip coating, drying the resulting film, and bonding the film on the surface of the intermediate layer, or yet alternatively by preparing a film of a polymer for the coating layer, bonding it to the surface of the intermediate layer, and coating the film with a solution of the medicine by dip coating, and drying the resulting film.

The method of dissolving the polymer for the coating layer or/and polymer for the intermediate layer in a solvent and applying the polymers in the solution state or the method of dissolving the polymer and the medicine for the coating layer in a solvent and applying the slution in the solution state do not restenose the advantageous effects of the present invention. Thus, various methods, including the method of dipping the stent main body in each solution and the method of applying each solution on the stent main body by spraying, may be used. The solvent for use is not particularly limited. A solvent having a desirable solubility is favorably used, and two or more solvents may be used as a mixed solvent, for adjustment of volatility and others. The solute concentration is also not particularly limited, and the optimal concentration is determined, taking into consideration the surface smoothness or the like of the intermediate layer and the coating layer. For adjustment of the surface smoothness, an excessive amount of the solution may be removed during the process of dissolving the polymer for the coating layer or/and the polymer for the intermediate layer in a solvent or/and after application thereof, or alternatively, during the process of dissolving the polymer for the coating layer and a medicine in a solvent and applying the polymer in the solution state or/and after application thereof. The solvent-removing means include vibration, rotation, evacuation, and the like, and these means may be used in combination.

### EXAMPLES

### (Example 1)

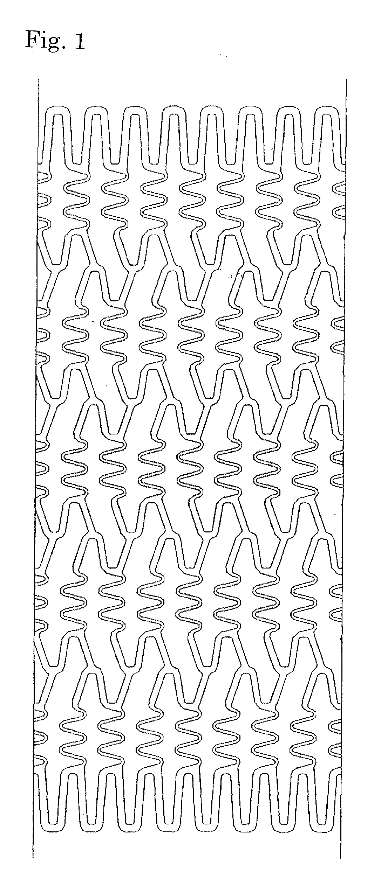

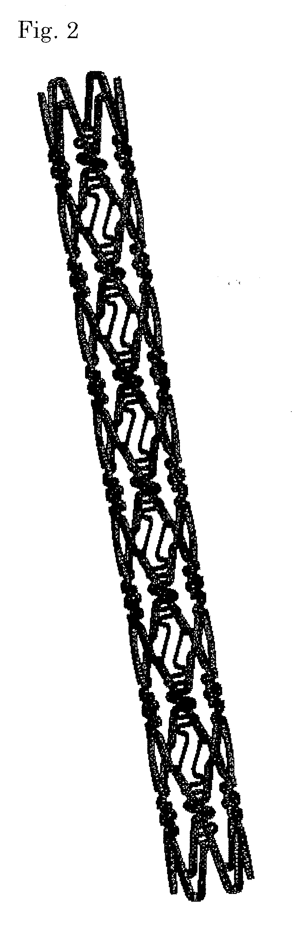

A stent main body was prepared by cutting a stainless steel tube (SUS316L) having an internal diameter of 1.50 mm and an external diameter of 1. 80 mm into the stent shape by laser cutting and polishing it electrolytically, similarly to the method normally practiced by those who are skilled in the art. Figure 1 is a development view of the stent, and Figure 2 is a schematic view thereof. The length of the stent was set to 13 mm, the thickness to 120 µm, and the nominal diameter after expansion to 3.5 mm. The stent is a so-called balloon expandable stent that is inflated and placed by using a balloon catheter equipped with a balloon in the region of the catheter close to the distal end. The balloon expandable stent, which is placed in the balloon region of the balloon catheter as it is contracted, is delivered to a desired site and inflated and placed there by expansion of the balloon.

A lactic acid-glycolic acid copolymer (product number: 85DG065, manufactured by Absorbable Polymers International, lactic acid/glycolic acid: 85/15, weight-average molecular weight: 85,000) was dissolved in chloroform (Wako Pure Chemical Industries Ltd.), to give a 0. 5 wt % solution. A stainless steel wire having a diameter of 100 µm was connected to one end of the stent, and the other end was connected to a stainless steel rod having a diameter of 2 mm. The stent was held in the direction perpendicular to the length direction, by connecting the stent-unconnected sided terminal of the stainless steel rod to a motor. The stent was rotated with the motor at a frequency of 100 rpm, and the solution prepared was sprayed on the stent by using a spray gun having a nozzle diameter of 0.3 mm. The distance between the nozzle of spray gun and the stent was 75 mm, and the air pressure during spraying was 0.15 MPa. The stent was dried after spraying under vacuum at room temperature for 1 hour. An intermediate layer having a weight of the glycolic lactate acid copolymer per unit length of the stent main body in the axial direction at 4 µg/mm (52 µg per stent) was formed while the spraying period was adjusted.

A lactic acid-glycolic acid copolymer (product number: RG502H, manufactured by Boehringer Ingelheim, lactic acid/glycolic acid: 50/50, weight-average molecular weight: 11,000) was dissolved in chloroform, to give a 0.5 wt % solution. A stainless steel wire having a diameter of 100 µm was connected to one end of the stent, and the other end was connected to a stainless steel having a diameter of 2 mm. The stent was held in the direction perpendicular to the length direction, by connecting the stent-unconnected sided terminal of the stainless steel rod to a motor. The stent was rotated with the motor at a frequency of 100 rpm, and the solution prepared was sprayed by using a spray gun having a nozzle diameter of 0.3 mm on the stent carrying the formed intermediate layer, allowing deposition of the solution. The distance between the nozzle of spray gun and the stent was 75 mm, and the air pressure during spraying was 0.15 MPa. The stent was dried after spraying under vacuum at room temperature for 1 hour. A coating layer having a weight of the glycolic lactate acid copolymer per unit length of the stent main body in the axial direction at 40 µg/mm (520 µg per stent) was formed while the spraying period was adjusted.

### (Example 2)

A stent having intermediate and coating layers was prepared in a similar manner to Example 1, except that the weight of the intermediate layer was changed to 2 µg/mm (26 µg per stent), the polymer for the coating layer was changed to another lactic acid-glycolic acid copolymer (product number: RG504H, manufactured by Boehringer Ingelheim, lactic acid/glycolic acid: 50/50, weight-average molecular weight; 50,000), and the weight thereof per unit length of the stent main body in the axial direction was changed to 35 µg/mm (455 µg per stent).

### (Example 3)

A stent having intermediate and coating layers was prepared in a similar manner to Example 1, except that the weight of the intermediate layer was changed to 2 µg/mm (26 µg per stent), the polymer for the coating layer was changed to poly-D,L-lactic acid (product number: R202H, manufactured by Boehringer Ingelheim, weight-average molecular weight: 12,000), and the weight thereof per unit length of the stent main body in the axial direction was changed to 50 µg/mm (650 µg per stent).

### (Example 4)

A stent having intermediate and coating layers was prepared in a similar manner to Example 1, except that the weight of the intermediate layer was changed to 4 µg/mm (52 µg per stent), the polymer for the coating layer was changed to poly-D,L-lactic acid (product number: R203H, manufactured by Boehringer Ingelheim, weight-average molecular weight: 22,000), and the weight thereof per unit length of the stent main body in the axial direction was changed to 42 µg/mm (546 µg per stent).

### (Example 5)

A stent having intermediate and coating layers was prepared in a similar manner to Example 1, except that the polymer for the intermediate layer was changed to poly-D, L-lactic acid (product number: 100D065, manufactured by Absorbable Polymers International, weight-average molecular weight: 83,000), the weight thereof per unit length of the stent main body in the axial direction was changed to 5 µg/mm (65 µg per stent), the polymer for the coating layer was changed to a lactic acid-glycolic acid copolymer (product number: RG502H, manufactured by Boehringer Ingelheim, lactic acid/glycolic acid: 50/50, weight-average molecular weight: 11,000), and the weight thereof per unit length of the stent main body in the axial direction was changed to 43 µg/mm (559 µg per stent).

### (Example 6)

A stent having intermediate and coating layers was prepared in a similar manner to Example 5, except that the weight of the intermediate layer was changed to 3 µg/mm (39 µg per stent), the polymer for the coating layer was changed to a lactic acid-glycolic acid copolymer (product number: RG504H, manufactured by Boehringer Ingelheim, lactic acid/glycolic acid: 50/50, weight-average molecular weight: 50,000), and the weight thereof per unit length of the stent main body in the axial direction was changed to 48 µg/mm (624 µg per stent).

### (Example 7)

A stent having intermediate and coating layers was prepared in a similar manner to Example 5, except that the weight of the intermediate layer was changed to 2 µg/mm (26 µg per stent), the polymer for the coating layer was changed to poly-D, L-lactic acid (product number: R202H, manufactured by Boehringer Ingelheim, weight-average molecular weight: 12,000), and the weight thereof per unit length of the stent main body in the axial direction was changed to 41 µg/mm (533 µg per stent).

### (Example 8)

A stent having intermediate and coating layers was prepared in a similar manner to Example 5, except that the weight of the intermediate layer was changed to 7 µg/mm (91 µg per stent), the polymer for the coating layer was changed to poly-D,L-lactic acid (product number: R203H, manufactured by Boehringer Ingelheim, weight-average molecular weight: 22,000), and the weight thereof per unit length of the stent main body in the axial direction was changed to 47 µg/mm (611 µg per stent).

### (Example 9)

As tent having intermediate and coating layers was prepared in a similar manner to Example 1, except that the polymer for the intermediate layer was changed to poly-L-lactic acid (product number: 100L105, manufactured by Absorbable Polymers International, weight-average molecular weight: 145,000), the weight thereof per unit length of the stent main body in the axial direction was changed to 3 µg/mm (per stent 39µg), the polymer for the coating layer was changed to a lactic acid-glycolic acid copolymer (product number: RG502H, manufactured by Boehringer Ingelheim, lactic acid/glycolic acid: 50/50, weight-average molecular weight: 11,000), and the weight thereof per unit length of the stent main body in the axial direction was changed to 30 µg/mm (390 µg per stent).

### (Example 10)

A stent having intermediate and coating layers was prepared in a similar manner to Example 9, except that the weight of the intermediate layer was changed to 5 µg/mm (65 µg per stent), the polymer for the coating layer was changed to a lactic acid-glycolic acid copolymer (product number: RG504H, manufactured by Boehringer Ingelheim, lactic acid/glycolic acid: 50/50, weight-average molecular weight: 50,000), and the weight thereof per unit length of the stent main body in the axial direction was changed to 48 µg/mm (624 µg per stent).

### (Example 11)

A stent having intermediate and coating layers was prepared in a similar manner to Example 9, except that the weight of the intermediate layer was changed to 4 µg/mm (52 µg per stent), the polymer for the coating layer was changed to poly-D,L-lactic acid (product number: R202H, manufactured by Boehringer Ingelheim, weight-average molecular weight: 12,000), and the weight thereof per unit length of the stent main body in the axial direction was changed to 50 µg/mm (650 µg per stent).

### (Example 12)

A stent having intermediate and coating layers was prepared in a similar manner to Example 9, except that the weight of the intermediate layer was changed to 5 µg/mm (65 µg per stent), the polymer for the coating layer was changed to poly-D,L-lactic acid (product number: R203H, manufactured by Boehringer Ingelheim, weight-average molecular weight 22,000), and the weight thereof per unit length of the stent main body in the axial direction was changed to 39 µg/mm (507 µg per stent).

### (Example 13)

A stent main body was prepared by cutting a stainless steel tube (SUS316L) having an internal diameter of 1.50 mm and an external diameter of 1.80 mm into the stent shape by laser cutting and polishing it electrolytically, similarly to the method normally practiced by those who are skilled in the art. Figure 1 is a development view of the stent, and Figure 2 is a schematic view thereof. The length of the stent was set to 13 mm, the thickness to 120 µm, and the nominal diameter after expansion to 3.5 mm. The stent is a so-called balloon expandable stent that is inflated and placed by using a balloon catheter having a balloon in the region of the catheter close to the distal end. The balloon expandable stent, which is placed in the balloon region of the balloon catheter as it is contracted, is delivered to a desired site and inflated and placed there by expansion of the balloon.

A lactic acid-glycolic acid copolymer (product number: 85DG065, manufactured by Absorbable Polymers International, lactic acid/glycolic acid: 85/15, weight-average molecular weight: 85,000) was dissolved in chloroform (Wako Pure Chemical Industries Ltd.), to give a 0. 5 wt % solution. A stainless steel wire having a diameter of 100 µm was connected to one end of the stent, and the other end was connected to a stainless steel rod having a diameter of 2 mm. The stent was held in the direction perpendicular to the length direction, by connecting the stent-unconnected sided terminal of the stainless steel rod to a motor. The stent was rotated with the motor at a frequency of 100 rpm, and the solution prepared was sprayed on the stent by using a spray gun having a nozzle diameter of 0.3 mm, allowing deposition of the solution. The distance between the nozzle of spray gun and the stent was 75 mm, and the air pressure during spraying was 0. 15 MPa. The stent was dried after spraying under vacuum at room temperature for 1 hour. An intermediate layer having a weight of the glycolic lactate acid copolymer per unit length of the stent main body in the axial direction at 3 µg/mm (39 µg per stent) was formed while the spraying period was adjusted.

A lactic acid-glycolic acid copolymer (product number: RG502H, manufactured by Boehringer Ingelheim, lactic acid/glycolic acid: 50/50, weight-average molecular weight: 11,000) and a medicine tacrolimus (developed by Fujisawa Pharmaceutical Co. , Ltd. and currently available from Astellas Pharma Inc.) were dissolved in chloroform, to give a mixed solution containing the lactic acid-glycolic acid copolymer at a concentration of 0.5 wt % and tacrolimus at a concentration of 0.19 wt %. A stainless steel wire having a diameter of 100 µm was connected to one end of the stent, and the other end was connected to a stainless steel rod having a diameter of 2 mm. The stent was held in the direction perpendicular to the length direction, by connecting the stent-unconnected sided terminal of the stainless steel rod to a motor. The stent was rotated with the motor at a frequency of 100 rpm, and the solution prepared was sprayed by using a spray gun having a nozzle diameter of 0.3 mm on the stent carrying the formed intermediate layer, allowing deposition of the solution. The distance between the nozzle of spray gun and the stent was 75 mm, and the air pressure during spraying was 0.15 MPa. The stent was dried after spraying under vacuum at room temperature for 1 hour. A coating layer having a weight of the glycolic lactate acid copolymer per unit length of the stent main body in the axial direction at 42 µg/mm (520 µg per stent) and a tacrolimus weight of 16 µg/mm (208 µg per stent) was formed while the spraying period was adjusted.

### (Example 14)

A stent having intermediate and coating layers was prepared in a similar manner to Example 13, except that the weight of the intermediate layer was changed to 7 µg/mm (91 µg per stent), the polymer for the coating layer was changed to a lactic acid-glycolic acid copolymer (product number: RG504H, manufactured by Boehringer Ingelheim, lactic acid/glycolic acid: 50/50, weight-average molecular weight: 50,000), and the weight of the lactic acid-glycolic acid copolymer per unit length of the stent main body in the axial direction was changed to 47 µg/mm (611 µg per stent) and the tacrolimus weight to 18 µg/mm (234 µg per stent).

### (Example 15)

A stent having intermediate and coating layers was prepared in a similar manner to Example 13, except that the polymer for the intermediate layer was changed to poly-D,L-lactic acid (product number: 100D065, manufactured by Absorbable Polymers International, weight-average molecular weight: 83,000), the weight of the poly-D,L-lactic acid per unit length of the stent main body in the axial direction was changed to 3 µg/mm (39 µg per stent), the polymer for the coating layer was changed to poly-D,L-lactic acid (product number: R202H, manufactured by Boehringer Ingelheim, weight-average molecular weight 12,000), and the weight of the poly-D,L-lactic acid per unit length of the stent main body in the axial direction was changed to 46 µg/mm (598 µg per stent) and the tacrolimus weight to 17 µg/mm (221 µg per stent).

### (Example 16)

A stent having intermediate and coating layers was prepared in a similar manner to Example 15, except that the weight of the intermediate layer was changed to 4 µg/mm (52 µg per stent), the polymer for the coating layer was changed to poly-D,L-lactic acid (product number: R203H, manufactured by Boehringer Ingelheim, weight-average molecular weight: 22,000), and the weight of the poly-D,L-lactic acid per unit length of the stent main body in the axial direction was changed to 42 µg/mm (546 µg per stent) and the tacrolimus weight to 16 µg/mm (208 µg per stent).

### (Example 17)

A stent having intermediate and coating layers was prepared in a similar manner to Example 13, except that the polymer for the intermediate layer was changed to poly-L-lactic acid (product number: 100L105, manufactured by Absorbable Polymers International, weight-average molecular weight 145,000), the weight of the poly-L-lactic acid per unit length of the stent main body in the axial direction was changed to 5 µg/mm (65 µg per stent), and the weight of the lactic acid-glycolic acid copolymer in the coating layer per unit length of the stent main body in the axial direction was changed to 40 µg/mm (520 µg per stent) and the tacrolimus weight to 15 µg/mm (195 µg per stent).

### (Example 18)

A stent having intermediate and coating layers was prepared in a similar manner to Example 13, except that the polymer for the intermediate layerwas changed to poly-L-lactic acid (product number: 100L105, manufactured by Absorbable Polymers International, weight-average molecular weight 145,000), the weight of the poly-L-lactic acid per unit length of the stent main body in the axial direction was changed to 4 µg/mm (52 µg per stent), the polymer for the coating layer was changed to a lactic acid-glycolic acid copolymer (product number: RG504H, manufactured by Boehringer Ingelheim, lactic acid/glycolic acid: 50/50, weight-average molecular weight: 50,000), the weight of the lactic acid-glycolic acid copolymer per unit length of the stent main body in the axial direction was changed to 39 µg/mm (507 µg per stent), and the medicine was changed to sirolimus (manufactured by SIGMA) and the sirolimus weight to 15 µg/mm (195 µg per stent).

### (Example 19)

A stent having intermediate and coating layers was prepared in a similar manner to Example 13, except that the polymer for the intermediate layer was changed to a lactic acid-glycolic acid copolymer (product number: 85DG065, manufactured by Absorbable Polymers International, lactic acid/glycolic acid: 85/15, weight-average molecular weight 85,000), the weight of the lactic acid-glycolic acid copolymer per unit length of the stent main body in the axial direction was changed to 6 µg/mm (78 µg per stent), the polymer for the coating layer was changed to a lactic acid-glycolic acid copolymer (product number: RG502H, lactic acid/glycolic acid: 50/50, manufactured by Boehringer Ingelheim, weight-average molecular weight 11,000), the weight of the lactic acid-glycolic acid copolymer per unit length of the stent main body in the axial direction was changed to to 37 µg/mm (481 µg per stent), the medicine was changed to cyclosporine (manufactured by Japan Ciba-Geigy K.K.), and the cyclosporine weight was changed to 14 µg/mm (182 µg per stent).

### (Comparative Example 1)

A stent having no intermediate but having a coating layer was prepared in a similar manner to Example 1, except that the polymer for the coating layer was changed to a lactic acid-glycolic acid copolymer (product number: RG502H, lactic acid/glycolic acid:50/50,manufactured by Boehringer Ingelheim, weight-average molecular weight:11,000),and the weight thereof per unit length of the stent main body in the axial direction was changed to 27 µg/mm (351 µg per stent)

### (Comparative Example 2)

A stent having no intermediate but having a coating layer was prepared in a similar manner to Comparative Example 1, except that the polymer for the coating layer was changed to a lactic acid-glycolic acid copolymer (product number: RG504H, lactic acid/glycolic acid: 50/50, manufactured by Boehringer Ingelheim, weight-averagemolecularweight: 50,000), and the weight thereof per unit length of the stent main body in the axial direction was changed to 45 µg/mm (585 µg per stent).

### (Comparative Example 3)

A stent having no intermediate but having a coating layer was prepared in a similar manner to Comparative Example 1, except that the polymer for the coating layer was changed to poly-D,L-lactic acid (product number: R202H, manufactured by Boehringer Ingelheim, weight-average molecular weight: 12,000), and the weight thereof per unit length of the stent main body in the axial direction was changed to 50 µg/mm (650 µg per stent).

### (Comparative Example 4)

A stent having no intermediate but having a coating layer was prepared in a similar manner to Comparative Example 1, except that the polymer for the coating layer was changed to poly-D,L-lactic acid (product number: R203H, manufactured by Boehringer Ingelheim, weight-average molecular weight 22,000), and the weight thereof per unit length of the stent main body in the axial direction was changed to 39 µg/mm (507 µg per stent).

### (Comparative Example 5)

A stent having no intermediate but having a coating layer was prepared in a similar manner to Example 13, except that the weight of the lactic acid-glycolic acid copolymer in the coating layer per unit length of the stent main body in the axial direction was changed to 40 µg/mm (520 µg per stent) and the tacrolimus weight to 15 µg/mm (195 µg per stent).

### (Comparative Example 6)

A stent having no intermediate but having a coating layer was prepared in a similar manner to Example 14, except that the weight of the lactic acid-glycolic acid copolymer in the coating layer per unit length of the stent main body in the axial direction was changed to 42 µg/mm (per stent 546µg), the medicine was changed to cyclosporine (manufactured by Japan Ciba-Geigy K.K.), and the cyclosporine weight was changed to 16 µg/mm (208 µg per stent).

### (Comparative Example 7)

A stent having no intermediate but having a coating layer was prepared in a similar manner to Example 15, except that the weight of the poly-D, L-lactic acid in the coating layer per unit length of the stent main body in the axial direction was changed to 39 µg/mm (507 µg per stent), the medicine was changed to sirolimus (manufactured by SIGMA), and the sirolimus weight was changed to 15 µg/mm (195 µg per stent).

### (Comparative Example 8)

A stent having no intermediate but having a coating layer was prepared in a similar manner to Example 16, except that the weight of the poly-D-L-lactic acid in the coating layer per unit length of the stent main body in the axial direction was changed to 38 µg/mm (494 µg per stent) and the tacrolimus weight to 14 µg/mm (182 µg per stent).

### (In-vitro evaluation experiment)

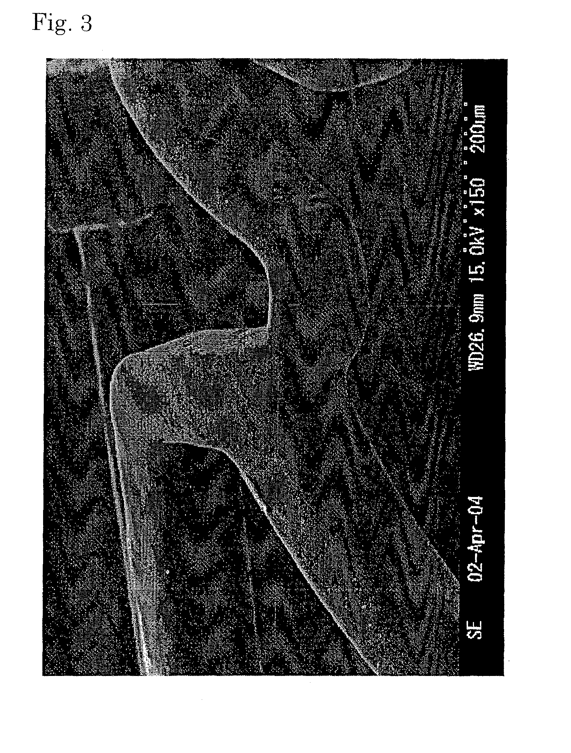

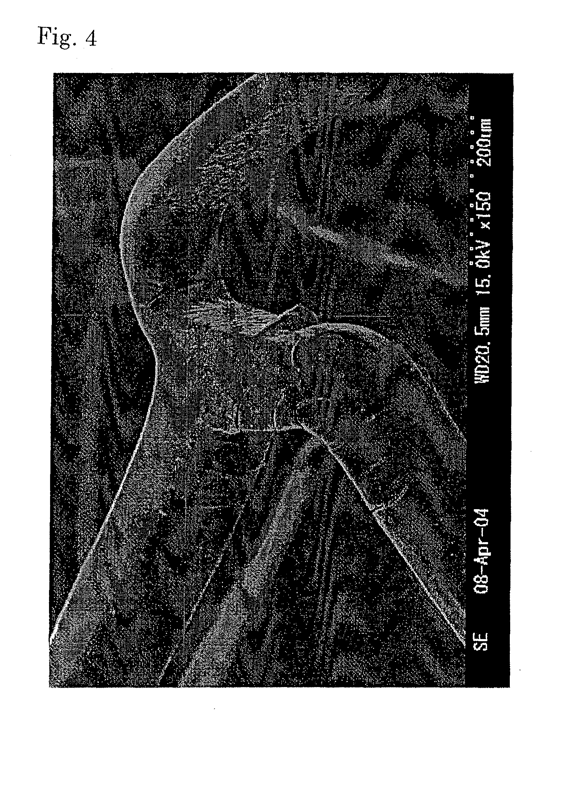

A PTCA balloon catheter containing a balloon of 3.5x15 mm in dimension was prepared, and the stent described above was mounted on the balloon region. The balloon was inflated in air at room temperature at 8 atm (810 kPa), allowing expansion of the stent. The balloon was deflated after 1 minute and separated from the stent. The expanded stent was fixed on the test-piece stage of an electron microscope and vapor-deposited with a Pt-Pd alloy, and the surface thereof was observed under a scanning electron microscope (S-3000N, manufactured by Hitachi High-Technologies Corp.). The frequency of cracking and exfoliation on the coated film was evaluated qualitatively, and the results are summarized in Tables 1 to 3. Figure 3 shows an SEM observation image of the sample in Example 3 and Figure 4 shows an SEM observation image of the sample in Comparative Example 6, as typical examples of the cracking and exfoliation of the coated film.

### (Evaluation results)

**[Table 1]**

| | Intermediate layer | | | Coating layer | | | | Evaluation result | |
|---|---|---|---|---|---|---|---|---|---|
| | Kind | Composition | Coating amount [µg/mm] | Kind | Composition | Coating amount [µg/mm] | Medicine coating amount [µg/mm] | Cracking | Exfoliation |
| Example 1 | PLGA | 85DG065 | 4 | PLGA | RG502H | 40 | - | ○ | ○ |
| Example 2 | PLGA | 85DG065 | 2 | PLGA | RG504H | 35 | - | ○ | ○ |
| Example 3 | PLGA | 85DG065 | 2 | PDLLA | R202H | 50 | - | ○ | ○ |
| Example 4 | PLGA | 85DG065 | 4 | PDLLA | R203H | 42 | - | ○ | ○ |
| Example 5 | PDLLA | 100D065 | 5 | PLGA | RG502H | 43 | - | ○ | ○ |
| Example 6 | PDLLA | 100D065 | 3 | PLGA | RG504H | 48 | - | ○ | ○ |
| Example 7 | PDLLA | 100D065 | 2 | PDLLA | R202H | 41 | - | ○ | ○ |
| Example 8 | PDLLA | 100D065 | 7 | PDLLA | R203H | 47 | - | ○ | ○ |
| Example 9 | PLLA | 100L105 | 3 | PLGA | RG502H | 30 | - | ○ | ○ |
| Example 10 | PLLA | 100L105 | 5 | PLGA | RG504H | 48 | - | ○ | ○ |

| | | | | | | | | | |
|---|---|---|---|---|---|---|---|---|---|
| PLGA: Lactic acid-glycolic acid copolymer PDLLA: Poly-D,L-lactic acid PLLA: Poly-L-lactic acid | | | | | | | | | |

**[Table 2]**

| | Intermediate layer | | | Coating layer | | | | Evaluation result | |
|---|---|---|---|---|---|---|---|---|---|
| | Kind | Composition | Coating amount [µg/mm] | Kind | Composition | Coating amount [µg/mm] | Medicine coating amount [µg/mm] | Cracking | Exfoliation |
| Example 11 | PLLA | 100L105 | 4 | PDLLA | R202H | 50 | - | ○ | ○ |
| Example 12 | PLLA | 100L105 | 5 | PDLLA | R203H | 39 | - | ○ | ○ |
| Example 13 | PLGA | 85DG065 | 3 | PLGA | RG502H | 42 | 16 | ○ | ○ |
| Example 14 | PLGA | 85DG065 | 7 | PLGA | RG504H | 47 | 18 | ○ | ○ |
| Example 15 | PDLLA | 100D065 | 3 | PDLLA | R202H | 46 | 17 | ○ | ○ |
| Example 16 | PDLLA | 100D065 | 4 | PDLLA | R203H | 42 | 16 | ○ | ○ |
| Example 17 | PLLA | 100L105 | 5 | PLGA | RG502H | 40 | 15 | ○ | ○ |
| Example 18 | PLLA | 100L105 | 4 | PLGA | RG504H | 39 | 15 | ○ | ○ |
| Example 19 | PLGA | 85DG065 | 6 | PLGA | RG502H | 37 | 14 | ○ | ○ |

| | | | | | | | | | |
|---|---|---|---|---|---|---|---|---|---|
| PLGA: Lactic acid-glycolic acid copolymer PDLLA: Poly-D,L-lactic acid PLLA: Poly-L-lactic acid | | | | | | | | | |

**[Table 3]**

| | Intermediate layer | | | Coating layer | | | | Evaluation result | |
|---|---|---|---|---|---|---|---|---|---|
| | Kind | Composition | Coating amount [µg/mm] | Kind | Composition | Coating amount [µg/mm] | Medicine coating amount [µg/mm] | Cracking | Exfoliation |
| Comparative Example 1 | - | - | - | PLGA | RG502H | 27 | - | × | ○ |
| Comparative Example 2 | - | - | - | PLGA | RG504H | 45 | - | × | ○ |
| Comparative Example 3 | - | - | - | PDLLA | R202H | 50 | - | × | × |
| Comparative Example 4 | - | - | - | PDLLA | R203H | 39 | - | × | ○ |
| Comparative Example 5 | - | - | - | PLGA | RG502H | 40 | 15 | × | × |
| Comparative Example 6 | - | - | - | PLGA | RG504H | 42 | 16 | × | × |
| Comparative Example 7 | - | - | - | PDLLA | R202H | 39 | 15 | × | × |
| Comparative Example 8 | - | - | - | PDLLA | R203H | 38 | 14 | × | × |

| | | | | | | | | | |
|---|---|---|---|---|---|---|---|---|---|
| PLGA: Lactic acid-glycolic acid copolymer PDLLA: Poly-D,L-lactic acid | | | | | | | | | |

As shown in Tables 1 to 3, the stents according to the invention having intermediate and coating layers obtained in Examples 1 to 19 were resistant to cracking and exfoliation of the coated film. On the other hand, the stents obtained in Comparative Examples 1 to 4 having only a coating layer and no intermediate layer showed cracking of the film, and the stent obtained in Comparative Example 3 even showed exfoliation. In addition, all of the stents obtained in Comparative Examples 5 to 8 having only a coating layer containing a medicine showed cracking and exfoliation of the coated film.

As described above, the stent for placement in body according to the present invention, which has a stent base material containing a material non-degradable in the body, a coating layer of polymer formed on at least part of the stent main body, and an intermediate layer of a polymer having a weight-average molecular weight of greater than that of the polymer above between the coating layer and the stent main body surface, is effectively resistant to exfoliation and cracking of the coating layer associated with stent expansion.

## Claims

1. An almost tube-shaped stent for placement in body expandable toward outside in the radial direction of the tube, **characterized by** including a stent main body containing a material non-degradable in the body as its base stent material, a coating layer containing a first polymer and a medicine, and an intermediate layer containing a second polymer having a weight-average molecular weight higher than that of the first polymer between the coating layer and the stent main body surface, wherein, the coating layer and the intermediate layer are present on at least part of the stent main body surface.

2. The stent for placement in body according to Claim 1, wherein the medicine is an immunosuppressive agent.

3. The stent for placement in body according to Claim 2, wherein the immunosuppressive agent is at least one compound selected from tacrolimus (FK506), cyclosporine, sirolimus (rapamycin), azathioprine, mycophenolate mofetil and the analogs thereof.

4. The stent for placement in body according to Claim 3, wherein the immunosuppressive agent is tacrolimus (FK506).

5. The stent for placement in body according to any one of Claims 1 to 4, wherein the first and second polymers are respectively first and second biodegradable polymers.

6. The stent for placement in body according to Claim 5, wherein each of the first and second biodegradable polymers is at least one polymer selected from polylactic acid, polyglycolic acid, and lactic acid-glycolic acid copolymers.

7. The stent for placement in body according to Claim 5, wherein the biodegradation period of the first biodegradable polymer is shorter than that of the second biodegradable polymer.

8. The stent for placement in body according to any one of Claims 1 to 4, wherein the weight-average molecular weight of the first polymer is 10,000 or more and 50,000 or less and that of the second polymer is 80,000 or more and 200,000 or less.
